(19) Europäisches Patentamt
European Patent Office
Office européen des brevets

(11) Numéro de publication: **0 080 600**
**B1**

(12) **FASCICULE DE BREVET EUROPEEN**

(45) Date de publication du fascicule du brevet: **19.06.85**

(51) Int. Cl.$^4$: **C 11 B 9/00,** A 61 K 7/46

(21) Numéro de dépôt: **82109838.1**

(22) Date de dépôt: **25.10.82**

# (54) Utilisation de 2-hydroxy-3,4,4-triméthyl-cyclopent-2-ène-1-one à titre d'ingrédient odoriférant.

(30) Priorité: **27.11.81 CH 7596/81**

(43) Date de publication de la demande:
**08.06.83 Bulletin 83/23**

(45) Mention de la délivrance du brevet:
**19.06.85 Bulletin 85/25**

(84) Etats contractants désignés:
**CH DE FR GB LI NL**

(56) Documents cités:
**FR-A-2 032 835**
**FR-A-2 351 078**

**S. ARCTANDER. "Perfume and flavor chemicals", vol. II, no. 1987, 1969, MONTCLAIR, N.J., USA**

(73) Titulaire: **FIRMENICH SA**
**1, route des Jeunes**
**CH-1211 Genève 8 (CH)**

(72) Inventeur: **Escher, Sina Dorothea**
**8, avenue du Lignon**
**CH-1219 Le Lignon (CH)**
Inventeur: **Morris, Anthony Francis**
**Route de Trélex**
**CH-1261 Gingins (CH)**

(74) Mandataire: **Salvadori, Giuseppe, Dr.**
**c/o Firmenich S.A. Case Postale 239**
**CH-1211 Genève 8 (CH)**

**EP 0 080 600 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

L'invention se rapporte au domaine de la parfumerie. Elle a trait, plus particulièrement, à l'emploi de 2-hydroxy-3,4,4,-triméthyl-cyclopent-2-ène-1-one de formule

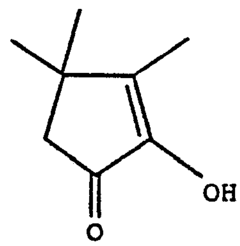

(I)

à titre d'ingrédient odoriférant. Elle est définie aux revendications.

La 2-hydroxy-3,4,4-triméthyl-cyclopent-2-ène-1-one (I) fait partie d'une série de dérivés de la cyclopentanone abondamment illustrée dans la littérature scientifique, plus particulièrement en ce qui concerne les dérivés mono-, di- ou tri-méthylés. Chose étonnante cependant, seul l'un d'entre-eux a retenu l'attention des parfumeurs. Il s'agit de la 2-hydroxy-3-méthyl-cyclopent-2-ène-1-one qui est en effet décrite comme possédant une odeur douce et puissante, de type caramel, épicé [voir à ce sujet S. Arctander, Perfume and Flavor Chemicals, Montclair N. J. 1969; section no. 1987]. Son emploi en parfumerie demeure toutefois fort restreint, l'intérêt essentiel de ce composé relevant du domaine des arômes.

En ce qui concerne les composés homologues diméthylés, seule leur utilité dans le domaine des arômes a été reconnue: la 2-hydroxy-3,4-diméthylcyclopent-2-ène-1-one, ainsi que les dérivés 3,5- et 5,5-diméthylés correspondants sont en effet appréciés pour leur note gustative de type caramel, noix, épicé.

La littérature est par contre totalement muette quant aux propriétés organoleptiques que pourraient présenter les composés homologues triméthylés telle la 2-hydroxy-3,4,4-triméthyl-cyclopent-2-ène-1-one (I) par exemple.

C'est dans ce contexte très particulier qu'il a été trouvé que la 2-hydroxy-3,4,4-triméthyl-cyclopent-2-ène-1-one (I), présentait de façon surprenante, d'intéressantes propriétés odoriférantes et qu'elle pouvait être ainsi d'un emploi fort avantageux pour les parfumeurs.

La 2-hydroxy-3,4,4-triméthyl-cyclopent-2-ène-1-one (I) se caractérise par son odeur originale que l'on peut qualifier de chaude, de type céleri, caramel, rappelant par certains côtés la composante aromatique de l'odeur du labdanum ciste ou de la myrrhe. Cette odeur puissante a notamment la particularité de se combiner harmonieusement avec toute une gamme d'odeurs usuelles, telles les notes boisées, épicées, chypre, animales, orentales, voire florales. De ce fait, la 2-hydroxy-3,4,4-triméthyl-cyclopent-2-ène-1-one (I) peut être avantageusement utilisée pour la préparation de compositions ou bases parfumantes destinées aussi bien à la parfumerie fine qu'au parfumage de produits techniques tels que savons, détergents, adoucissants pour textiles par exemple, produits cosmétiques ou produits d'entretien, cires ou laques par exemple.

Au vu de sa note olfactive tout à la fois chaude et épicée, la 2-hydroxy-3,4,4-triméthyl-cyclopent-2-ène-1-one (I) peut être en outre avantageusement utilisée lors de la reconstitution de certaines huiles essentielles ou résines telles par exemple la patchouli, le fenugrec, la myrrhe, l'opoponax ou l'oliban.

Des effets olfactifs tels que définis ci-dessus peuvent être obtenus par l'emploi dudit composé à titre d'ingrédient odoriférant unique, par exemple sous forme de solution dans les solvants conventionnels tels l'alcool éthylique, le phtalate diéthylique, le dipropylène-glycol ou le citrate d'éthyle, ou encore sous forme de mélange avec les ingrédients odoriférants usuels en parfumerie, dans ce cas sous forme de coeurs ou bases pour parfums par exemple.

L'effet olfactif obtenu par l'emploi de la 2-hydroxy-3,4,4-triméthyl-cyclopent-2-ène-1-one (I) dépend notamment de la concentration et de la nature des coingrédients de la base ou composition à laquelle on l'a incorporée. Lors de la préparation de compositions parfumantes par exemple, on peut obtenir des effets olfactifs intéressants par l'emploi de quantités de l'ordre de 0,01% (poids) déjà. Les effets les plus caractéristiques s'obtiennent de préférence par l'emploi de quantités comprises entre 0,05 et 5% environ du poids de la composition considérée, des quantités supérieures à 5% pouvant être également envisagées, notamment lorsque des effets plus particuliers sont recherchés.

La 2-hydroxy-3,4,4-triméthyl-cyclopent-2-ène-1-one (I) est un composé connu: elle peut être obtenue selon les prescriptions de la littérature [voir à ce sujet Gazz. Chim. Ital, *101*, 225 (1971)]. La qualité olfactive du produit ainsi préparé (F. 83—85°C) convient parfaitement à l'usage du composé (I) tel que défini plus haut.

L'exemple ci-après illustrera l'invention de façon plus détaillée, sans pour autant la limiter.

Exemple

On a premièrement préparé une composition parfumante de base de type chypre à partir des ingrédients suivants:

| Ingrédients | Parties en poids |
|---|---|
| Benjoin Siam | 200 |
| Essence de bergamote synth. | 3100 |
| Base CYCLOSIA ® 1) | 500 |
| EXALTEX ® x) | 1000 |
| IRALIA ® 1) | 1500 |
| Mousse de chêne abs. à 50% * | 1000 |
| VERTOFIX ® coeur 2) | 2000 |
| Essence de rose synth. | 500 |
| Total | 9800 |

* dans le phtalate diétylique
1) origine FIRMENICH SA — Genève, Suisse
2) origine International Flavors & Fragrances Inc. — New York, USA

A l'aide de la base ci-dessus et des 3 composés A, B et C mentionnés ci-après, on a préparé les compositions parfumantes suivantes:

Composé A: 2-hydroxy-3-méthyl-cyclopent-2-ène-1-one [voir S. Arctander, op. cit.)
Composé B: 2-hydroxy-3-diméthyl-cyclopent-2-ène-1-one [voir Tetrahedron *19,* 2039 (1963)]
Composé C: 2-hydroxy-3,4,4-triméthyl-cyclopent-2-ène-1-one.

| Composition | A | B | C | D |
|---|---|---|---|---|
| Base chypre | 9800 | 9800 | 9800 | 9800 |
| Composé A | 2 | — | — | — |
| Composé B | — | 2 | — | — |
| Composé C | — | — | 2 | — |
| Phtalate diéthylique | 198 | 198 | 198 | 200 |
| Total | 10000 | 10000 | 10000 | 10000 |

Après avoir été diluée à environ 10% dans l'alcool éthylique à 95%, chacune des compositions parfumanates ainsi préparées a été soumise à évaluation. Le groupe d'experts consulté s'est prononcé comme suit:

Composition A: odeur plaisante, pas de grand changement par rapport à D
Composition B: effet plus marqué, note noix-caramel; rappelle également le céleri, le fenugrec; genre condiments
Composition C: effet très net, note chaude rappelant la livêche, la myrrhe, l'opopnax et la labdanum. Plus riche et plus élégant que D.

**Revendications**

1. Utilisation de 2-hydroxy-3,4,4-triméthyl-cyclopent-2-ène-1-one à titre d'ingrédient odoriférant pour la préparation de parfums, bases ou compositions parfumantes ou de produits parfumés.
2. Utilisation de 2-hydroxy-3,4,4-triméthyl-cyclopent-2-ène-1-one selon la revendication 1 à l'état pur ou en combinaison avec un ou plusieurs ingrédients odoriférants additionnels, un solvant ou un support.

**Patentansprüche**

1. Verwendung von 2-Hydroxy-3,4,4-trimethyl-cyclopent-2-en-1-on als geruchstragender Bestandteil zur Herstellung von Parfümen, Basen oder Parfüm-Kompositionen oder von parfümierten Produkten.

2. Verwendung von 2-Hydroxy-3,4,4-trimethyl-cyclopent-2-en-1-on gemäss Anspruch 1, worin es in reiner Form oder in Kombination mit einem oder mehreren Bestandteilen, einem Lösungsmittel oder einem Träger, verwendet wird.

**Claims**

1. Utilization of 2-hydroxy-3,4,4-trimethyl-cyclopent-2-en-1-one as fragrance ingredient for the preparation of perfumes, of base- or fragrance-compositions or of perfumed products.

2. Utilization according to claim 1 of 2-hydroxy-3,4,4-trimethyl-cyclopent-2-en-1-one in pure state or in combination with one or several additional ingredients, a solvent or a support.